# EUROPEAN PATENT SPECIFICATION

(11) **EP 2 768 482 B1**
(45) Date of publication and mention of the grant of the patent: **04.12.2019**
(21) Application number: 11808952.3
(22) Date of filing: 21.10.2011
(51) Int. Cl.: A61K 51/12, A61K 45/06, A61K 31/137, A61K 9/00, A61K 9/06

(54) **ANTI-TUMORAL COMPOUND AND RELATIVE PRODUCTION PROCESS**
ANTITUMORVERBINDUNG UND HERSTELLUNGSVERFAHREN DAFÜR
COMPOSÉ ANTITUMORAL ET SON PROCÉDÉ DE PRODUCTION

(43) Date of publication of application: 27.08.2014
(73) Proprietor: Betaglue Technologies S.P.A., 20121 Milano (IT)
(72) Inventor: DI CAPUA, Giulia, I-80122 Napoli (IT)
(74) Representative: Cooley (UK) LLP
(86) International application number: PCT/IT2011/000354
(87) International publication number: WO 2013/057747

(56) References cited:
- EP-A1- 0 167 263
- WO-A2-2005/079757
- US-A- 5 597 578
- US-A1- 2002 168 319

## Description

The present invention relates to the field of therapeutic drugs and medicines for antitumor treatments.

More in detail, the present invention concerns an anti-tumoral compound for the localized treatment of neoplastic pathologies of malignant kind that cannot be excised by surgery, or with a high risk of local recurrence.

It is well known that in presence of neoplastic pathologies of malignant kind like, e.g., hepatocellular carcinoma, colorectal cancer or breast cancer, the removal of the tumoral mass is the only therapeutic treatment that guarantees the long term survival of the patient.

It is furthermore known that, at the light of the present medical knowledge, not all neoplastic pathologies of malignant kind may be removed by surgery, as said therapeutic method is tightly depending on the patient's clinical condition, on the localization of the neoplasias and on the morphologic features of the tumoral mass to be removed.

The removal of a neoplastic mass by surgical resection cannot be performed in the following cases:
- if in the patient's body there is a non neoplastic pathology (comorbidity) involving the organ attacked by the tumoral mass, or the anatomic region comprising the same;
- if the removal of the tumoral mass determines the alteration of the functionality of the treated organ;
- if the removal of he tumoral mass determines aesthetic or functional modifications such as to permanently change the lifestyle of the patient.

Furthermore, the surgical removal of a tumoral mass can not be performed without the certainty of obtaining a resection margin of healthy tissue of at least one centimetre from the infiltration limit of the tumoral cells, as it has been widely shown that resection margins less than one centimetre favour the recurrence of neoplastic pathologies of malignant kind in the original surgical site.

It is known that therapeutic methods alternative to surgical resection of a tumoral mass usually comprise:
- chemotherapy or radiotherapy treatments, systemic and/or locoregional;
- intra-arterial treatments like, e.g., chemoembolization, chemoperfusion or radioembolization;
- localized ablative treatments, obtained by means of quick freezing (cryoablation), chemical desiccation (alcohol ablation), or necrosis of tumor cells induced by hyperthermia (ablation by means of laser, microwaves, radio frequency, etc.).

It is further known that said methods have important and numerous limits or side effects, as:
- the high toxicity of the antineoplastic substances used in the treatments like chemotherapy and radiotherapy makes a long term application of said therapies impossible;
- the quick freezing of the tumoral tissues, or the necrosis induced by hypothermia thereof, causes breaks and bleedings in the cell structure of the treated organs;
- the desiccation of the tumoral tissues, obtained by the localized injection of alcohol, determines an incomplete necrosis of the same due to a non uniform distribution of said substance through the tissues.

Furthermore, none of said therapies proves to be radical for the purpose of allowing a long term survival of the patient.

In the pharmaceutical field, the method consisting in the inclusion of one or more active principles in a viscous material, functioning as a carrier of the same, for obtaining medical compounds allowing the localized treatment of different pathologies, is well known.

Among the many typologies of compounds that may be commonly obtained with said method, those with anti-inflammatory, antimicrobial, antihemorrhagic, dermoregenerating, analgesic, disinfectant and other action will be reported as an example.

EP 0 167 263 A1 discloses an antineoplastic composition comprising a proteinaceous glue dispersed in an aqueous means, a cis-platinum based cytotoxic antineoplastic substance and a substance acting as vasoconstrictor. US 2002/168319 and WO 2005/079757 both disclose tissue glue comprising a particulate radiotherapeutic agent.

Therefore, it is the aim of the present invention to obtain a compound for antitumor use that may be applied for the local treatment of neoplastic pathologies of malignant kind that cannot be surgically treated or showing a high risk of local recurrence by the inclusion, in a viscous material functioning as a carrier, of a substance having appropriate neoplastic capacities and functioning as a therapeutic agent.

It is furthermore the aim of the present invention to overcome some of the problems and of the limits deriving from the use of therapeutic methods commonly used for the treatment of neoplastic pathologies of malignant kind which cannot be treated surgically, or having a high risk of local recurrence.

The aim set forth is reached by means of an anti-tumoral compound, according to the independent claim 1 or 2.

Further features of the anti-tumoral compound according to the present invention are described in the dependent claims.

A process for the production of said anti-tumoral compound is also the object of the present invention.

The compound according to the present invention has many and important advantages:
- it may be given at an intra-tumoral level, for the purpose of determining the complete necrosis of the tumoral tissues around the inoculation area, thus allowing the treatment of tumoral masses of malignant kind that cannot be surgically removed;
- it may be applied as a filling material of surgical wounds deriving from a surgical resection, or from ablative treatments, of tumoral masses of malignant kind, for the purpose of determining the complete necrosis of possible residual tumoral cells localized along the edges of said wounds;
- it determines a greater efficiency of antineoplastic substances, producing a favourable increase of the administration period and of the dosage of said substances at a local level, due to the concentration of the same inside the treated tumoral masses, or inside said surgical wounds deriving from surgical removal, or from ablative treatments, of the same;
- it prevents the free diffusion of said antineoplastic substances in the body of the patient, consequently limiting the systemic exposure to the toxic components of the same;
- it allows overcoming of the side effects deriving from the application of the therapeutic methods substitute for the surgical resection of the tumoral masses of malignant kind;
- besides, the inclusion of neoplastic substances into a material having viscous structure limits the exposure of the medical staff to the toxic components of said antineoplastic substances when the drug is given to the patient.

The anti-tumoral compound according to the present invention is based, as said before, on the principle of including a substance with suitable antineoplastic capacities into a special material with a viscous structure, arranged for functioning as a carrier for said substance.

Special biocompatible microspheres have been labelled with a radioactive high energy isotope for therapeutic use, then including the radiolabelled microspheres into a quick solidification haemostatic gel, thus obtaining a compound for antitumor action that may be directly inoculable inside tumoral masses that cannot be surgically treated, or that may be applied as a filling material in surgical wounds deriving from surgical resection of said tumoral masses.

Actually, histologic examinations have shown that said compound induces a favourable necrosis of the neoplastic cells in the area corresponding to inoculation into the tumoral mass of interest, or near the application area in the wound deriving from surgical resection of said tumoral mass.

The necrosis of the tumoral cells of interest is induced by means of an appropriate internal radiotherapy with electrons (IRE), determined by the localized emission of radioactive particles by said radiolabelled microspheres.

Laboratory examinations, carried out in a gamma camera and with PET/CT, have surprisingly shown that the compound spreads in an almost homogeneous manner inside the tumoral mass into which it has been inoculated, or inside the surgical wound onto which it has been applied, due to the viscous structure of the haemostatic gel forming the matrix thereof, and that such homogeneous diffusion is highly functional to the necrosis of the neoplastic cells localized in such areas.

The same examinations have also surprisingly shown that, if a haemostatic gel with a sufficient solidification speed is used as a carrier of said radiolabelled microspheres, the possible dispersion of said microspheres in the patient's body - potentially deriving from factors like gravity, their diffusion in the blood and/or lymphatic vessels, the presence of serosanguineous collections and other - is strongly limited, consequently avoiding any possible side effect deriving from possible dispersion of radioactive material in the patient himself.

Finally, further laboratory examinations have shown that the dispersion of the radiolabelled microspheres in the patient's body may be further limited including a substance in the compound arranged for determining a temporary vasoconstriction of the tissues surrounding the inoculation or application point of the same.

According to the present invention, and in a not restrictive exemplification, an anti-tumoral compound for local use according to the present invention mainly consists of:
- a quick solidification, bicomponent haemostatic gel resulting from the localized injection of two distinct chemical compounds, dosed in adequate ratio of quantity, one of which is fibrinogen and aprotinin-based, and the other one of which is thrombin and calcium chloride-based;
- a solution of biocompatible microspheres, comprising diameters between 20 and 60 µm, arranged for being included in the chemical solutions forming above mentioned haemostatic gel, labelled with a high energy radioactive isotope for therapeutic use, like e.g. the radioactive yttrium (⁹⁰Y), such as to determine the necrosis of the tumoral tissues around the inoculation or application area of the anti-tumoral compound;
- an epinephrine-based solution, also arranged for being included in the chemical solutions forming said haemostatic gel, such as to determine a temporary vasoconstriction effect to the tissues around the inoculation or application area of said anti-tumoral compound.

According to the present invention, above described anti-tumoral compound is arranged for being directly inoculated into tumoral masses of malignant kind that cannot be surgically treated, for determining the complete necrosis of the neoplastic cells that are near the inoculation point thereof.

It is also arranged for being applied as a filling material in surgical wounds deriving from surgical resection, or from ablative treatments, of tumoral masses of malignant kind, for determining the complete necrosis of possible residual neoplastic cells along the edges of the wounds, so that said edges are surrounded by at least one centimetre of healthy tissue thus reducing the risk of a possible recurrence of the neoplasia in the original site.

Said anti-tumoral compound induces the complete necrosis of the neoplastic cells near its inoculation or application point by means of an adequate internal radiotherapy with electrons (IRE) determined by the localized emission of radioactive particles by the radiolabelled microspheres comprised in the haemostatic gel forming the matrix thereof. Moreover, the complete necrosis of the neoplastic cells is favoured by the homogeneous distribution of the compound inside the tumoral mass into which it is inoculated or inside the surgical wound onto which it is applied, which is made possible due to the viscosity of the haemostatic gel forming the matrix thereof.

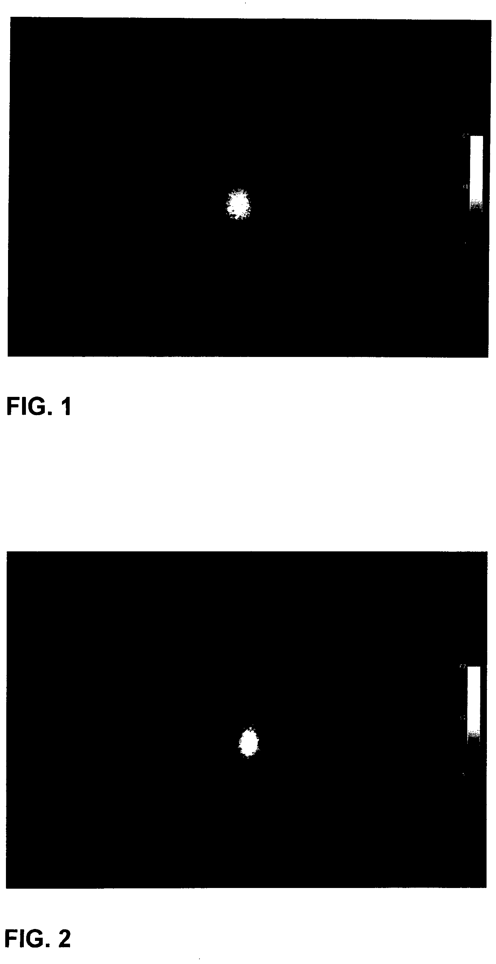

Evidence of the homogeneous distribution of the compound in cavity is given in enclosed figures 1 and 2 wherein, by a gamma-camera in front and back projection, the distribution of 1 mCi (37 MBq) of microspheres is shown, said microspheres being radiolabelled with the radioactive yttrium isotope (⁹⁰Y) and comprised in a quick solidification bicomponent haemostatic gel, inside a 2,5 ml syringe barrel.

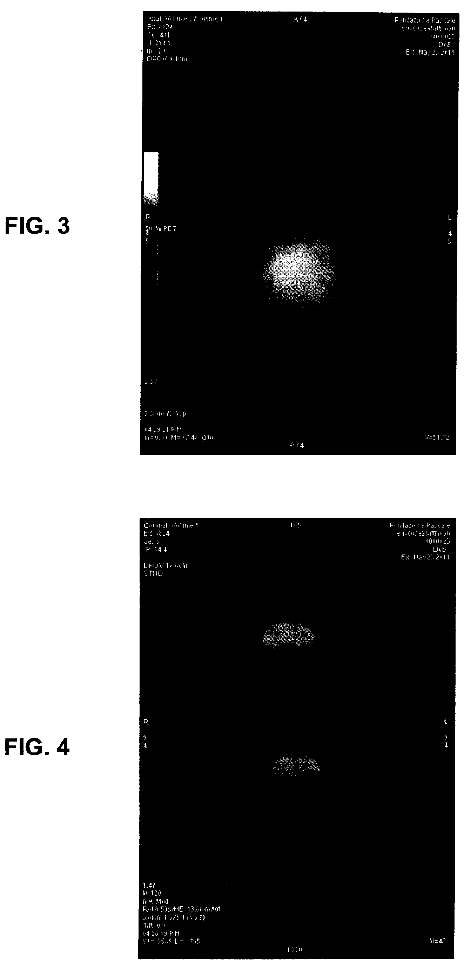

Figures 3 and 4 show - respectively by means of PET/CT and CT - the distribution of 340-370 microspheres MBq, radiolabelled with the radioactive yttrium isotope (⁹⁰Y) and comprised in a quick solidification, bicomponent haemostatic gel, inside 5 ml volume plastic spheres.

Such figures also show that the anti-tumoral compound does not reveal any dissociation between the solutions and the solidified gel, and that said gel does not reveal alterations of its distribution and of its solidification near the cavity of interest.

The quick solidification of the haemostatic gel, forming the matrix of the anti-tumoral compound, determines the confinement of the radiolabelled microspheres inside the inoculation or application site of said compound, advantageously producing a prolongation of its administration time and a consequent increase of its local dosage, deriving from the strong concentration of said microspheres inside each single tumoral mass, or of each single surgery wound treated.

The quick solidification of said haemostatic gel also limits the dispersion of the radiolabelled microspheres in the patient's body, potentially caused by factors like the gravity, the diffusion in blood and lymphatic vessels, the presence of serosanguineous collections and other, consequently avoiding the setting in of common side effects due to the dispersion of radioactive material in the patient himself.

Said haemostatic gel is obtained by the local injection of a couple of distinct chemical solutions, according to appropriate ratios, one of which fibrinogen and aprotinin-based, with a dosage of 1ml, and the other one thrombin and calcium chloride-based, with a dosage of 0,75 ml.

The injection of the two chemical solutions forming said haemostatic gel by means of a coaxial catheter with double lumen, e.g. of the kind disclosed in patent application MI2009A000969 of 3rd June 2009.

Said gel comprises a solution of biocompatible microspheres labelled with a radioactive isotope for therapeutic use, characterized in the emission of high energy electrons and with a penetration power in the tissue >5 mm, equal to 500-625 cellular layers, and preferably represented by the yttrium radioactive isotope (⁹⁰Y).

With a dosage of 0,25 ml, said solution allows to include inside said haemostatic gel a quantity of biocompatible microspheres equal to 30-60x10⁶ with a diameter comprised between 20 and 60 µm and labelled with the radioactive yttrium isotope (⁹⁰Y), having an average life of 64,1 hours and a tissue penetration coefficient of 11 mm, with an average penetration of the same of 2,5 mm, such as to determine the complete necrosis of the cancer tissues surrounding the inoculation or application area of the examined anti-tumoral compound.

Said microspheres, which are unassimilable by the human body, release 94% of the radioactivity of the yttrium isotope (⁹⁰Y) contained therein, in a time period superior to the average physical and biological life of said isotope, evaluated in 10-13 days from the moment of inoculation or application of said anti-tumoral compound.

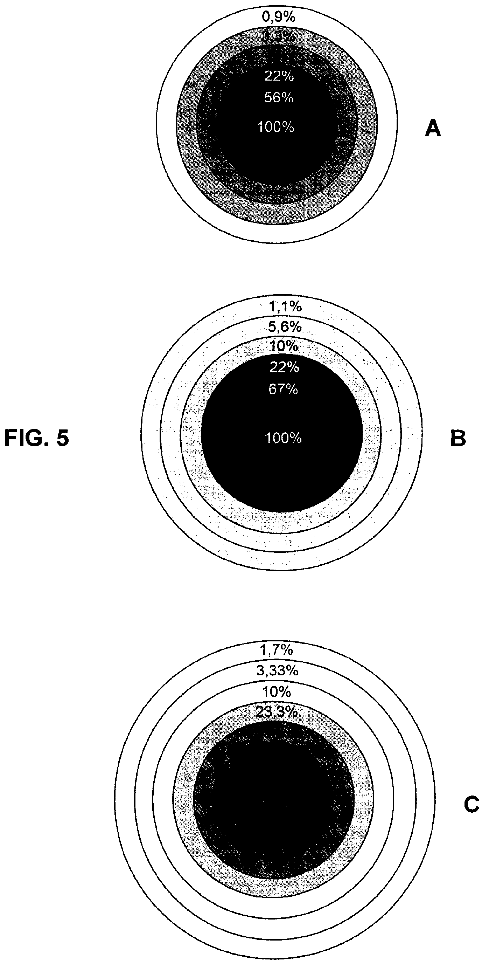

Figure 5 shows in a graph the results of the measurements, done through a gamma camera and PEC/CT, of the absorption percentage of the radioactivity released by said microspheres in contiguous shells with dimensions of 1 mm, near cavities of different volumes A, B, C, respectively equal to 0,5ml, 4,2 ml and 11,5 ml.

Figure 6 shows in a graph the results of the measurements of the activity percentage of the yttrium radioactive isotope (⁹⁰Y), according to its distance from the centre of said reference cavities.

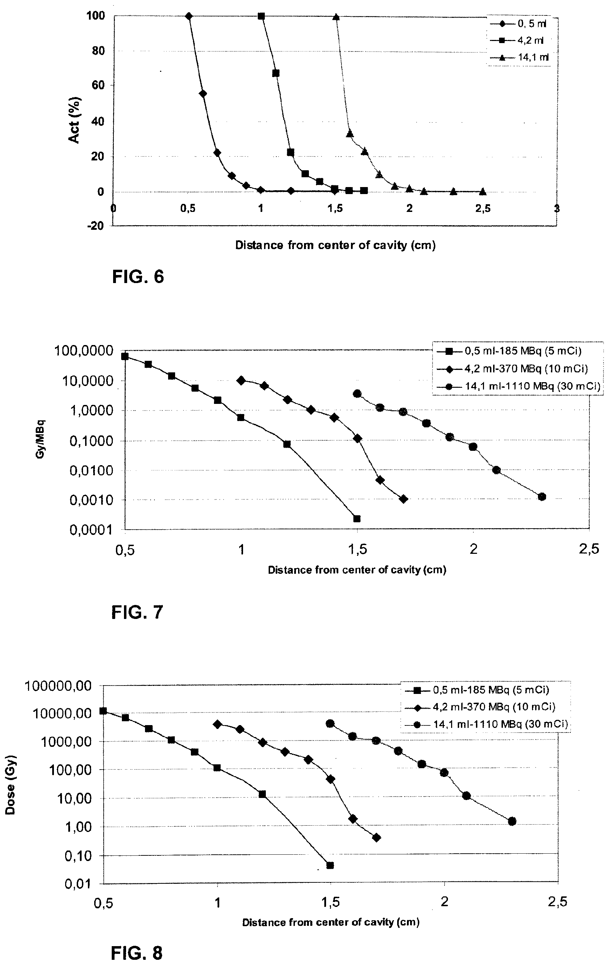

The data shown in the graphs in figures 7 and 8 have been processed from the activity values of the yttrium isotope (⁹⁰Y), released for each reference cavity, and said figures respectively show:
- the entity of radiation dosage (Gy/MBq) absorbed per unity, in adjoining shells of cavities of different dimensions, with reference volumes respectively equal to 0,5 ml, 4,2 ml and 11,5 ml;
- entities of radiation dosage (Gy) absorbed in a whole, in adjoining shells of cavities of different dimensions, with reference volumes respectively equal to 0,5 ml, 4,2 m and 11,5 ml.

Following table 1, on the other hand, reports the results of the measurements of the radiation dosage absorbed by wounds of different dimension, with the reference radius respectively equal to 0,5 cm, 1 cm and 1,5 cm.

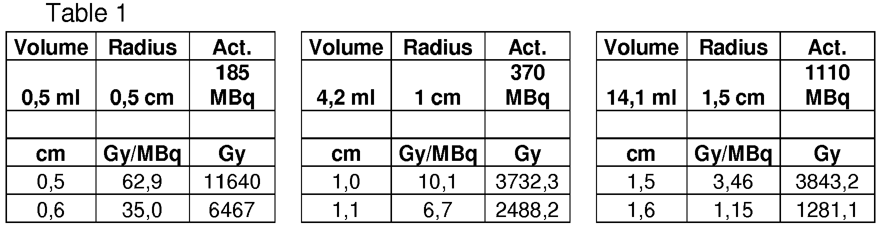

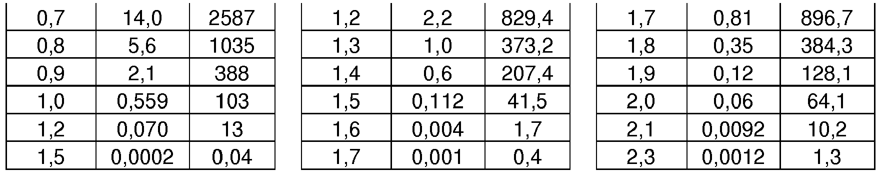

The results of the measurements, and of the processing deriving therefrom, confirm the strong concentration of the radiotherapy action of the radiolabelled microspheres near the inoculation or application point of the examined anti-tumoral compound.

Besides, the results confirm the limited dispersion of radioactive elements in the areas around the inoculation or application point of said compound, and consequently their limited dispersion in the patient's body.

As an alternative to above mentioned biocompatible microspheres, common antineoplastic substances with chemotherapy action may be comprised inside said haemostatic gel forming the matrix of said anti-tumoral compound.

Furthermore, an epinephrine solution is included inside said haemostatic gel, with a dosage equal to 0,1 ml/mg, arranged for determining a temporary vasoconstriction of the tissues around the inoculation or application area of the compound.

Said solution determines an advantageous increase of the haemostatic effect already given by the gel forming the matrix of said compound and, strongly reducing the bleeding of the tissues treated, it further limits the dispersion of the antineoplastic substances in the patient's body and thus avoids the arising of common side effects deriving from the systemic exposure to the toxic components of the same.

The particular features of the anti-tumoral compound above described, like the highly localized administration and the limited dispersion of the antineoplastic substances contained therein, allow an advantageous use of said compound in the field of the therapeutic treatment of tumoral masses localized in the area of the head and in the brain tissues, in the area of the neck, of the chest cavity, of the abdominal cavity and in the area of the retroperitoneal space, in the area of the pelvis as well as in the bones and in the soft tissues in general.

Said features also allow the use of said compound as an independent therapeutic treatment, suitable for allowing the treatment of tumoral masses that cannot be surgically removed, or as a therapeutic treatment complementary to surgical resection, or to alternative surgical techniques, suitable for maximizing the efficiency of those therapies and reducing the risk of neoplastic recurrences in the original site.

Best results have been obtained, with respect to similar substances, with the use of TISSUCOL (BAXTER) or Beriplast P (Nycomed), as a biological glue of SIR (SIRTeX) spheres as biocompatible microsphere solution; and of epinephrine (adrenaline) belonging to the adrenergic activity catecholamines of A and B type, as a local vasoconstrictor agent on the smooth muscles.

Best results have been obtained also with the use of the biological glue Coseal (BAXTER), a surgical sealant resulting from the localized injection of two different synthetic polyethylene glycols (PEGs), of a diluted solution of hydrochloric acid and of a solution of sodium phosphate/sodium carbonate.

During administration, said polyethylene glycols and said solutions form a hydrogel that adheres to the tissues and covalently binds itself to the same.

The pharmacologic behaviour of the anti-tumoral compound according to the present invention has been examined in different species, making use of animals with spontaneous or implanted neoplasias.

The results of said studies have shown - in imaging mode - that following to the administration of the compound in the wound, no loss of radiolabelled microspheres occurs in the tissues around the area of its inoculation or application.

By means of autoradiography it has been proved, in particular, that the dose injected in the tested animals remains active inside the solidified gel until 13 days after its injection.

Histological examination in a number of pigs has shown the necrosis of all neoplastic cells comprised in the area of administration of the anti-tumoral compound and said area varies between 0,6 and 3,2 mm.

The dose given to the animals has been considered tumoricidal for wounds between 3 and 22 cc, and no radioactivity has been found in the blood of the same.

Besides, different dosages of yttrium radioactive isotope (⁹⁰Y) have been evaluated, according to the volume of the residual surgical cavity to be filled up or to the dimensions of the wound to be treated, as appropriately summed up in following Table 2.

**Table 2**

| ***Volume of the remaining cavity or of the wound (cc)*** | ***Activity of the ⁹⁰Y isotope (MBq)*** | ***Volume of injected medicine (ml)*** |
|---|---|---|
| 1 | 259-370 (7-10 mCi) | 3 |
| 3 | 444-555 (12-15 mCi) | 4 |
| 5 | 592-740 (16-20 mCi) | 6 |
| 10 | 1110-1480 (30-40 mCi) | 12 |
| 20 | 2220-2960 (60-80 mCi) | 24 |
| >20 | 3000 | 24 |

The precondition for the dosimetric evaluation is that the yttrium radioactive isotope (⁹⁰Y) is homogeneously distributed inside the residual surgical cavity and inside the solidified gel forming the matrix of said compound, obtained through the co-injection of TISSUCOL (BAXTER), or Beriplast P (Nycomed), and epinephrine.

The different components of the haemostatic gel forming the matrix of the anti-tumoral compound have been given through a 2,5 ml syringe by means of a slow infusion carried out through a special coaxial catheter with double lumen.

The ratio between the fibrinogen-aprotinin solution, the thrombin-calcium chloride solution and the microsphere SIR solution was respectively of 1 ml:0,75 ml:0,25 ml.

In this preparation, the activity of the yttrium radioactive isotope (⁹⁰Y) has been of 22 MBq (0,6mCi).

The exposure of the staff to the components of the compound has been evaluated by means of a dosimetric thermoluminescence detector (TDL), because the preparation and the administration procedure of the compound must be considered as potentially dangerous.

Such exposure has been estimated supposing to manage a 3 GBq device with a time for preparing the dose of 30 minutes (for technical staff) and suggesting an average dose of 2 GBq in the patients and a time for the injection of the dose of 20 minutes (for medical staff).

During work, the detector has been placed near the pelvis, on the collar of the T-shirt and on the fingers.

The results are summed up in following table 3.

**Table 3**

| ***Technical staff*** | ***Surface dosage (0,07 mm)*** | ***Deep dosage (10 mm)*** |
|---|---|---|
| Pelvis mSv (mrem) | 0,027 (2,7) | 0,003 (0,3) |
| Eyes mSv (mrem) | 0,026 (2,6) | 0,004 (0,4) |
| Hands mSv (mrem) | 0,35 (35) | |
| | | |

| ***Medical staff*** | ***Surface dosage (0,07 mm)*** | ***Deep dosage (10 mm)*** |
|---|---|---|
| Pelvis mSv (mrem) | 0,038 (3,8) | 0,004 (0,4) |
| Eyes mSv (mrem) | 0,12 (12) | 0,054 (5,4) |
| Hands mSv (mrem) | 0,35 (35) | |

In a non-limiting example, the production process of the anti-tumoral compound according to the present invention comprises:
- a preparation phase of a chemical fibrinogen and aprotinin-based injectable compound;
- a preparation phase of a chemical thrombin and calcium chloride-based injectable compound;
- a phase of pre-dosing of said chemical compounds, inside special disposable syringes, and the subsequent low temperature storage of the same;
- a warming phase of said compounds, when they are to be used, up to the temperature of 37°C;
- a first phase of adding said compounds carried out by means of a solution of biocompatible microspheres, labelled with a high energy isotope for therapeutic use, like the yttrium radioactive isotope (⁹⁰Y);
- a second phase of adding said compounds, carried out by means of an epinephrine-based solution;
- an administration phase of equivalent doses of said compounds, determining the localized formation of a haemostatic gel comprising above listed adding substances, carried out by means of a special coaxial catheter with double lumen.

In a possible embodiment, the process for the production of said anti-tumoral compound comprises:
- a preparation phase of a chemical injectable compound consisting of a synthetic polyethylene glycol and of a diluted solution of hydrochloric acid;
- a preparation phase of a chemical injectable compound consisting of a synthetic polyethylene glycol and of a solution of sodium phosphate/sodium carbonate;
- a pre-dosing phase of said chemical compounds inside special disposable syringes, and the subsequent low temperature storage of the same;
- a warming phase of said compounds at the moment of use up to the temperature of 37°C;
- a first phase of adding said compounds, carried out by means of a solution of biocompatible microspheres, labelled with a high energy isotope for therapeutic use, like the yttrium radioactive isotope (⁹⁰Y);
- a second phase of adding said compounds, carried out by means of an epinephrine-based solution;
- an administration phase of equivalent doses of said compounds, determining the localized formation of a hydrogel comprising above mentioned adding substances, carried out by means of a special coaxial catheter with double lumen.
   disposable syringes, and the subsequent low temperature storage of the same;
- a warming phase of said compounds at the moment of use up to the temperature of 37°C;
- a first phase of adding said compounds, carried out by means of a solution of biocompatible microspheres, labelled with a high energy isotope for therapeutic use, like the yttrium radioactive isotope (⁹⁰Y);
- a second phase of adding said compounds, carried out by means of an epinephrine-based solution;
- an administration phase of equivalent doses of said compounds, determining the localized formation of a hydrogel comprising above mentioned adding substances, carried out by means of a special coaxial catheter with double lumen.

## Claims

1. An anti-tumoral compound, comprising:
a biologic glue comprising two injectable distinct chemical compounds;
an antineoplastic substance comprising a solution of biocompatible microspheres with radiotherapy action as a therapeutic agent, wherein said biocompatible microspheres comprise diameters between 20 and 60 µm and are labelled with a radioactive isotope for therapeutic use having a high energy electrons emission and with a tissue penetration capacity of >5 mm, equal to 500-625 cellular layers;
wherein said biologic glue is a haemostatic gel, arranged for solidifying, so as to keep the antineoplastic substance in loco.

2. An anti-tumoral compound, comprising:
a biologic glue comprising two injectable distinct chemical compounds;
an antineoplastic substance comprising a solution of biocompatible microspheres with radiotherapy action as a therapeutic agent, wherein said biocompatible microspheres comprise diameters between 20 and 60 µm and are labelled with a radioactive isotope for therapeutic use having a high energy electrons emission and with a tissue penetration capacity of >5 mm, equal to 500-625 cellular layers;
wherein said biologic glue is a hydrogel, arranged for solidifying, so as to keep the antineoplastic substance in loco.

3. An anti-tumoral compound according to claim 1 or 2, **characterized in that** said injectable biologic glue is arranged for being given to patients by means of a double lumen catheter.

4. An anti-tumoral compound according to claim 1 or 2, **characterized in that** said isotope contained inside said biocompatible microspheres is the yttrium radioactive isotope (90Y).

5. A process for the production of a compound for antitumor use,
**characterized in that** it comprises:
a preparation phase of a chemical fibrinogen and aprotinin-based injectable compound;
a preparation phase of a chemical thrombin and calcium chloride- based injectable compound;
a phase of pre-dosing of said chemical compounds, inside special disposable syringes, and the subsequent low temperature storage of the same;
a warming phase of said compounds, when they are to be used, up to the temperature of 37°C;
a first phase of adding said compounds carried out by means of a solution of biocompatible microspheres, comprising diameters between 20 and 60 µm and labelled with a high energy yttrium radioactive isotope (⁹⁰Y);
a second phase of adding said compounds;
a phase of using a coaxial catheter with double lumen to dose in adequate ratio of quantity said compounds, determining the localized formation of a haemostatic gel comprising above listed adding substances.

6. A process for the production of a compound for antitumor use, **characterized in that** it comprises:
a preparation phase of a chemical injectable compound consisting of a synthetic polyethylene glycol and of a diluted solution of hydrochloric acid;
a preparation phase of a chemical injectable compound consisting of a synthetic polyethylene glycol and of a solution of sodium phosphate/sodium carbonate;
a pre-dosing phase of said chemical compounds inside special disposable syringes, and the subsequent low temperature storage of the same;
a warming phase of said compounds at the moment of use up to the temperature of 37°C;
a first phase of adding said compounds, carried out by means of a solution of biocompatible microspheres, comprising diameters between 20 and 60 µm and labelled with a high energy yttrium radioactive isotope (⁹⁰Y); a second phase of adding said compounds;
a phase of using a coaxial catheter with double lumen to dose in adequate ratio of quantity said compounds, determining the localized formation of a hydrogel comprising above mentioned adding substances.

## Patentansprüche

1. Antitumorverbindung, die Folgendes umfasst:
einen biologischen Klebstoff, der zwei verschiedene injizierbare chemische Verbindungen umfasst;
eine antineoplastische Substanz, die eine Lösung von biokompatiblen Mikrosphären mit Strahlentherapiewirkung als therapeutisches Mittel umfasst, wobei die genannten biokompatiblen Mikrosphären Durchmesser zwischen 20 und 60 µm haben und mit einem radioaktiven Isotop für den therapeutischen Gebrauch mit einer hochenergetischen Elektronenemission und einer Gewebeeindringkapazität von >5 mm, entsprechend 500-625 Zellschichten, markiert sind;
wobei der genannte biologische Klebstoff ein hämostatisches Gel ist, das zum Verfestigen ausgelegt ist, um die antineoplastische Substanz in loco zu halten.

2. Antitumorverbindung, die Folgendes umfasst:
einen biologischen Klebstoff, der zwei verschiedene injizierbare chemische Verbindungen umfasst;
eine antineoplastische Substanz, die eine Lösung von biokompatiblen Mikrosphären mit Strahlentherapiewirkung als therapeutisches Mittel umfasst, wobei die genannten biokompatiblen Mikrosphären Durchmesser zwischen 20 und 60 µm haben und mit einem radioaktiven Isotop für den therapeutischen Gebrauch mit einer hochenergetischen Elektronenemission und einer Gewebeeindringkapazität von >5 mm, entsprechend 500-625 Zellschichten, markiert sind;
wobei der genannte biologische Klebstoff ein Hydrogel ist, das zum Verfestigen ausgelegt ist, um die antineoplastische Substanz in loco zu halten.

3. Antitumorverbindung nach Anspruch 1 oder 2, **dadurch gekennzeichnet, dass** der genannte injizierbare biologische Klebstoff so ausgelegt ist, dass er Patienten über einen Doppellumenkatheter verabreicht wird.

4. Antitumorverbindung nach Anspruch 1 oder 2, **dadurch gekennzeichnet, dass** das in den genannten biokompatiblen Mikrosphären enthaltene Isotop das radioaktive Yttriumisotop (⁹⁰Y) ist.

5. Verfahren zur Herstellung einer Verbindung für den Antitumorgebrauch, **dadurch gekennzeichnet, dass** es Folgendes beinhaltet:
eine Präparationsphase einer injizierbaren chemischen Verbindung auf der Basis von Fibrinogen und Aprotinin:
eine Präparationsphase einer injizierbaren chemischen Verbindung auf der Basis von Thrombin und Calciumchlorid;
eine Phase der Vordosierung der genannten chemischen Verbindungen in speziellen Einwegspritzen und deren anschließende Lagerung bei niedriger Temperatur;
eine Erwärmungsphase der genannten Verbindungen, wenn sie verwendet werden sollen, bis zu einer Temperatur von 37°C;
eine erste Phase der Zugabe der genannten Verbindungen, die mit Hilfe einer Lösung von biokompatiblen Mikrosphären erfolgt, die Durchmesser zwischen 20 und 60 µm haben und mit einem hochenergetischen radioaktiven Yttriumisotop (⁹⁰Y) markiert sind;
eine zweite Phase der Zugabe der genannten Verbindungen;
eine Phase der Verwendung eines koaxialen Katheters mit doppeltem Lumen, um die genannten Verbindungen in einem angemessenen Mengenverhältnis zu dosieren, wobei die lokalisierte Bildung eines hämostatischen Gels bestimmt wird, das die oben aufgeführten Zugabesubstanzen umfasst.

6. Verfahren zur Herstellung einer Verbindung für den Antitumorgebrauch, **dadurch gekennzeichnet, dass** es Folgendes beinhaltet:
eine Präparationsphase einer injizierbaren chemischen Verbindung, die aus einem synthetischen Polyethylenglykol und einer verdünnten Lösung von Chlorwasserstoffsäure besteht;
eine Präparationsphase einer injizierbaren chemischen Verbindung, die aus einem synthetischen Polyethylenglykol und einer Lösung von Natriumphosphat/Natriumcarbonat besteht;
eine Phase der Vordosierung der genannten chemischen Verbindungen in speziellen Einwegspritzen und deren anschließende Lagerung bei niedriger Temperatur;
eine Erwärmungsphase der genannten Verbindungen beim Gebrauch bis zu einer Temperatur von 37°C;
eine erste Phase der Zugabe der genannten Verbindungen, die mit Hilfe einer Lösung von biokompatiblen Mikrosphären erfolgt, die Durchmesser zwischen 20 und 60 µm umfassen und mit einem hochenergetischen radioaktiven Yttriumisotop (⁹⁰Y) markiert sind;
eine zweite Phase der Zugabe der genannten Verbindungen;
eine Phase der Verwendung eines koaxialen Katheters mit doppeltem Lumen, um die genannten Verbindungen in einem angemessenen Mengenverhältnis zu dosieren, wobei die lokalisierte Bildung eines Hydrogels bestimmt wird, das die oben erwähnten Zugabesubstanzen umfasst.

## Revendications

1. Composé antitumoral, comprenant :
une colle biologique comprenant deux composés chimiques injectables distincts ;
une substance antinéoplasique comprenant une solution de microsphères biocompatibles dotées d'une action radiothérapeutique en tant qu'agent thérapeutique, dans laquelle lesdites microsphères biocompatibles ont des diamètres compris entre 20 et 60 µm et
sont marquées avec un radio-isotope à usage thérapeutique ayant une émission d'électrons de haute énergie et une capacité de pénétration dans un tissu > 5 mm, égale à 500-625 couches cellulaires ;
dans lequel ladite colle biologique est un gel hémostatique conçu pour se solidifier afin de maintenir la substance antinéoplasique en place.

2. Composé antitumoral, comprenant :
une colle biologique comprenant deux composés
chimiques injectables distincts ;
une substance antinéoplasique comprenant une solution de microsphères biocompatibles dotées d'une action radiothérapeutique en tant qu'agent thérapeutique, dans laquelle lesdites microsphères biocompatibles ont des diamètres compris entre 20 et 60 µm et
sont marquées avec un radio-isotope à usage thérapeutique ayant une émission d'électrons de haute énergie et une capacité de pénétration dans un tissu > 5 mm, égale à 500-625 couches cellulaires ;
dans lequel ladite colle biologique est un hydrogel conçu pour se solidifier afin de maintenir la substance antinéoplasique en place.

3. Composé antitumoral selon la revendication 1 ou 2, **caractérisé en ce que** ladite colle biologique injectable est conçue pour être administrée à des patients au moyen d'un cathéter à double lumière.

4. Composé antitumoral selon la revendication 1 ou 2, **caractérisé en ce que** ledit isotope contenu à l'intérieur desdites microsphères biocompatibles est le radio-isotope d'yttrium (⁹⁰Y).

5. Procédé de production d'un composé à usage antitumoral, **caractérisé en ce qu'**il comprend :
une phase de préparation d'un composé chimique injectable à base de fibrinogène et d'aprotinine ;
une phase de préparation d'un composé chimique injectable à base de thrombine et de chlorure de calcium ;
une phase de prédosage desdits composés chimiques dans des seringues jetables spéciales, et leur stockage subséquent à basse température ;
une phase de chauffage desdits composés, lorsqu'ils sont destinés à être utilisés jusqu'à une température de 37 °C ;
une première phase d'ajout desdits composés effectuée au moyen d'une solution de microsphères biocompatibles, ayant des diamètres compris entre 20 et 60 µm et
marquées avec un radio-isotope d'yttrium de haute énergie (⁹⁰Y);
une seconde phase d'ajout desdits composés ;
une phase d'utilisation d'un cathéter coaxial à double lumière pour doser dans un rapport adéquat la quantité desdits composés, déterminant la formation localisée d'un gel hémostatique comprenant les substances d'ajout précitées.

6. Procédé de production d'un composé à usage antitumoral, **caractérisé en ce qu'**il comprend :
une phase de préparation d'un composé chimique injectable consistant en un polyéthylène glycol synthétique et une solution diluée d'acide chlorhydrique ;
une phase de préparation d'un composé chimique injectable consistant en un polyéthylène glycol synthétique et une solution de phosphate de sodium / carbonate de sodium ;
une phase de prédosage desdits composés chimiques dans des seringues jetables spéciales, et leur stockage subséquent à basse température ;
une phase de chauffage desdits composés au moment de l'utilisation jusqu'à la température de 37°C ;
une première phase d'ajout desdits composés, effectuée au moyen d'une solution de microsphères biocompatibles ayant des diamètres compris entre 20 et 60 µm
et marquées avec un radio-isotope d'yttrium de haute énergie (⁹⁰Y) ; une seconde phase d'ajout desdits composés ;
une phase d'utilisation d'un cathéter coaxial à double lumière pour doser dans un rapport adéquat la quantité desdits composés, déterminant la formation localisée d'un hydrogel comprenant les substances d'ajout précitées.
